(19) Europäisches Patentamt
European Patent Office
Office européen des brevets

(11) Veröffentlichungsnummer: **0 457 015 B1**

# (12) EUROPÄISCHE PATENTSCHRIFT

(45) Veröffentlichungstag der Patentschrift: **15.03.95**

(51) Int. Cl.$^{6}$: **A61K 31/53**

(21) Anmeldenummer: **91105456.7**

(22) Anmeldetag: **06.04.91**

Die Akte enthält technische Angaben, die nach dem Eingang der Anmeldung eingereicht wurden und die nicht in dieser Patentschrift enthalten sind.

## (54) Verwendung von substituierten 1,2,4-Triazindionen zur Bekämpfung von parasitären Protozoen.

(30) Priorität: **17.05.90 DE 4015835**
**22.09.90 DE 4030042**

(43) Veröffentlichungstag der Anmeldung:
**21.11.91 Patentblatt 91/47**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI LU NL SE**

(56) Entgegenhaltungen:
**EP-A- 0 170 316**
**EP-A- 0 330 041**
**EP-A- 0 353 526**
**EP-A- 0 377 903**
**EP-A- 0 377 904**
**DE-A- 2 532 363**

(73) Patentinhaber: **BAYER AG**
**D-51368 Leverkusen (DE)**

(72) Erfinder: **Lindner, Werner, Dr.**
**Märchenstrasse 39**
**W-5000 Köln 80 (DE)**
Erfinder: **Haberkorn, Axel, Prof. Dr.**
**Fuhlrottstrasse 99**
**W-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 457 015 B1

## Beschreibung

Die vorliegende Erfindung betrifft die Verwendung von substituierten 1,2,4-Triazindionen als Mittel zur Bekämpfung parasitärer Protozoen und insbesondere Coccidien sowie Fisch- und Insektenparasiten.

Die Verwendung bestimmter substituierter 1,2,4-Triazindione zur Bekämpfung von Coccidien ist bekannt. Die Wirkung dieser Verbindungen befriedigt jedoch nicht in jedem Fall (vgl. EP-OS 330 041, EP-OS 353 526).

Es wurde gefunden daß die substituierten 1,2,4-Triazindione der allgemeinen Formel

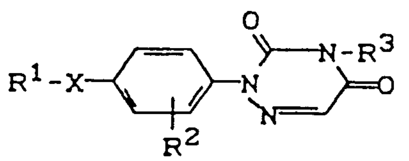

I

in welcher

- $R^1$ für gegebenenfalls durch Halogen, Alkyl, Cyano, Nitro, Alkoxy, Alkylthio, Halogenalkyl, Halogenalkoxy, Halogenalkylthio, Halogensulfonyl, Halogenalkylsulfinyl, Amino, Alkylamino, Halogenalkylamino, Acylamino substituiertes Pyridyl, Pyrazinyl oder Pirimidinyl steht,
- X für O, S, SO, $SO_2$ oder $-CR^4(CN)-$ steht,
- $R^2$ für Wasserstoff einen oder mehrere, gleiche oder verschiedene Reste der Gruppe Wasserstoff, Halogen, Nitro, Alkyl, Alkoxy, Alkylthio, Halogenalkyl, Halogenalkoxy, Alkylthio, Halogenalkylthio, steht,
- $R^3$ für Wasserstoff, Alkyl, Alkenyl, Alkinyl, Aralkyl steht,
- $R^4$ für Wasserstoff oder Alkyl steht,

sich hervorragend zur Bekämpfung parasitärer Protozoen eignen.

Die Verbindungen der Formel I sind Gegenstand einer älteren Anmeldung der Anmelderin (DE-OS 3 900 373). Sie dienen dort zur Herstellung der entsprechenden hydrierten Verbindungen,

Substituierte 1,2,4-Triazindione der allgemeinen Formel I

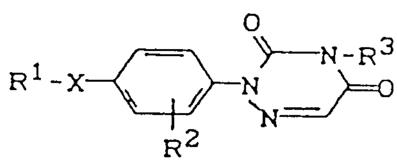

in welcher

X, $R^1$, $R^2$, $R^3$ die oben angegebene Bedeutung haben,

können hergestellt werden, indem man

a) Verbindungen der Formel Ia

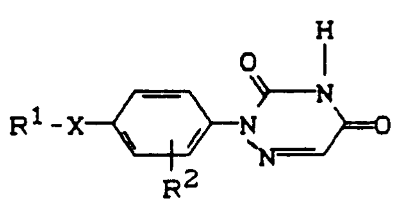

Ia

in welcher

X, $R^1$ und $R^2$ die oben angegebene Bedeutung haben,

mit Verbindungen der Formel II

$R^3$-B II

in welcher

$R^3$ die oben angegebenen Bedeutungen hat und

B für Halogen, $OSO_2$-Alkyl, -O-$SO_2$-Aryl, -O-$SO_2$-Halogenalkyl steht,

umsetzt, oder

b) daß man Verbindungen der Formel III

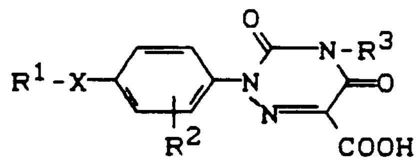

III

in welcher

X, $R^1$, $R^2$, $R^3$ die oben angegebenen Bedeutungen haben,

durch Erhitzen decarboxyliert oder

c) daß man Verbindungen der Formel IV

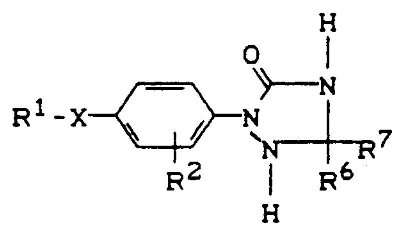

IV

in welcher

| | |
|---|---|
| X | für O, S, -$CR^4$(CN)- steht, |
| $R^1$, $R^2$ | die oben angegebene Bedeutung haben, |
| $R^4$ | für Wasserstoff oder Alkyl steht, |
| $R^6$ und $R^7$ | für gegebenenfalls substituiertes Alkyl stehen, |

mit Glyoxylsäure der Formel X

CHO-COOH X

in Gegenwart von anorganischen oder organischen Säuren umsetzt, oder

d) daß man Verbindungen der Formel V

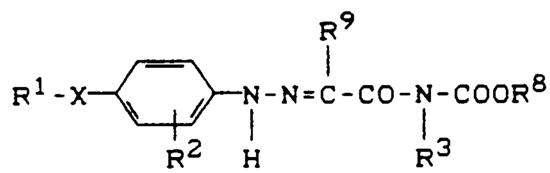

V

in welcher

| | |
|---|---|
| X | für O, S oder -$CR^4$(CN)- steht, |
| $R^1$, $R^2$, $R^3$, $R^4$ | die oben angegebene Bedeutung haben, und |
| $R^8$ | für Alkyl oder gegebenenfalls substituiertes Aryl steht, |
| $R^9$ | für H steht, |

in Gegenwart von Basen erhitzt, oder

e) daß man Verbindungen der Formel VI

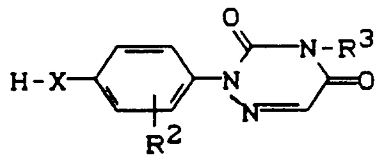

VI

in welcher

X für O oder S steht,

$R^2$, $R^3$ die oben angegebene Bedeutung haben,

mit Verbindungen der Formel VII

$R^1$-A VII

in welcher

$R^1$ die oben angegebene Bedeutung hat und

A für die Reste Halogen, O-$SO_2$-Alkyl, -O-$SO_2$-Halogenalkyl, -O-$SO_2$-Aryl, -S-Alkyl, -$SO_2$-Alkyl oder $SO_2$-Halogenalkyl steht,

umsetzt.

Die Verbindungen der Formeln VII, IV, V, VI sind zum Teil bekannt aus DOS 3 805 660 oder Gegenstand einer älteren noch nicht veröffentlichten Anmeldung der Anmelderin (Deutsche Anmeldung P 39 00373.6).

Bevorzugt verwendete Verbindungen der Formel I sind Verbindungen in denen

$R^1$ für gegebenenfalls durch Halogen, Alkyl, Cyano, Nitro, Alkoxy, Alkylthio, Halogenalkyl, Halogenalkoxy, Halogenalkylthio, Halogenalkylsulfonyl, Halogenalkylsulfinyl, Amino, Alkylamino, Halogenalkylamino, Acylamino substituiertes Pyrimidinyl, Pyridinyl, Pyrazinyl steht,

X für O, S oder -CH(CN)- steht,

$R^2$ für Halogen oder $C_{1-6}$-Alkyl steht,

$R^3$ für Wasserstoff oder $C_1$-$C_4$-Alkyl, insbesondere Methyl, steht.

Besonders bevorzugt sind Verbindungen der Formel I in welcher

X für O oder -CH(CN)- steht,

$R^1$ für Pyridinyl oder Pyrimidinyl steht, die gegebenenfalls durch $C_{1-4}$-Alkyl, insbesondere Methyl, $C_{1-4}$-Halogenalkyl insbesondere Trifluormethyl, Halogen, insbesondere Chlor, Brom, Fluor, Nitro, CN, $C_{1-4}$-Alkoxy insbesondere Methoxy, $C_{1-4}$-Halogenalkoxy insbesondere Trifluormethoxy, $C_{1-4}$-Alkylthio insbesondere Methylthio, $C_{1-4}$-Halogenalkylthio insbesondere Trifluormethylthio, $C_{1-4}$ Halogenalkylsulfonyl, insbesondere Trifluormethylsulfonyl, $C_{1-4}$-Halogenalkylsulfinyl, insbesondere Trifluormethylsulfinyl, Amino, $C_{1-4}$-Alkylamino, $C_{1-4}$-Halogenalkylamino, Acylamino insbesondere Acetylamino substituiert sind, steht,

$R^2$ für einen oder mehrere Reste der Gruppe Wasserstoff oder Halogen insbesondere Chlor, Brom, $C_{1-4}$-Alkyl insbesondere Methyl, 1-5-Halogen($C_{1-4}$)-alkyl, insbesondere Trifluormethyl steht,

$R^3$ für Wasserstoff oder Methyl steht,

Ganz besonders bevorzugt sind Verbindungen der Formel I in welcher

X für O steht,

$R^1$ für gegebenenfalls durch Nitro, Chlor oder Methyl oder Trifluormethyl substituiertes Pyridinyl steht,

$R^2$ für einen oder mehrere Reste der Gruppe Wasserstoff, Methyl oder Chlor steht,

$R^3$ für Wasserstoff oder Methyl steht.

Als Einzelverbindungen seien genannt:

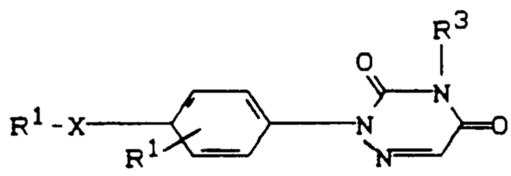

| $R^1$ | X | $R^2$ | $R^3$ |
|---|---|---|---|
| 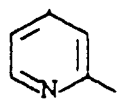 | CHCN | 3,5-$Cl_2$ | H |
| 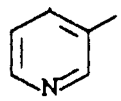 | CHCN | 3,5-$Cl_2$ | H |
| 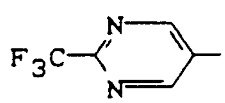 | O | 3,5-$Cl_2$ | H |
| 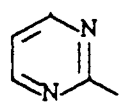 | O | 3,5-$Cl_2$ | H |
| 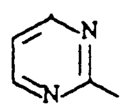 | O | 3,5-$CH_3$ | H |
| 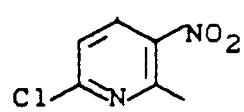 | O | 3,5-$Cl_2$ | H |
| 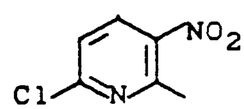 | O | 3,5-$CH_3$ | H |
| 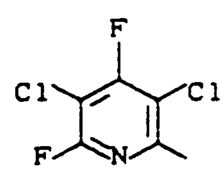 | O | 3,5-$Cl_2$ | H |

| $R^1$ | X | $R^2$ | $R^3$ |
|---|---|---|---|
| 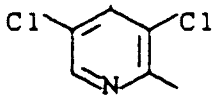 | O | $3,5\text{-}Cl_2$ | H |
| 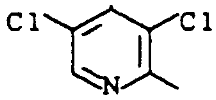 | O | $3,5\text{-}CH_3$ | H |
| 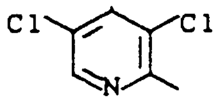 | O | $3,5\text{-}(CH_3)_2$ | H |
| 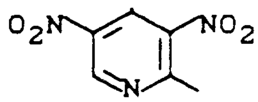 | O | $3,5\text{-}Cl_2$ | H |
| 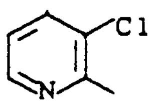 | O | $3,5\text{-}Cl_2$ | H |
| 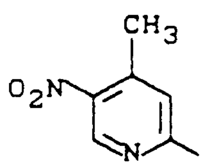 | O | $3,5\text{-}Cl_2$ | H |
| 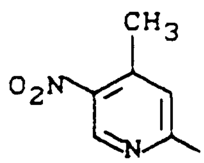 | O | $3,5\text{-}CH_3$ | H |
| 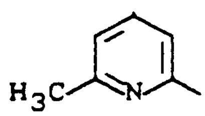 | O | $3,5\text{-}Cl_2$ | H |
| 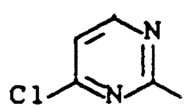 | O | $3,5\text{-}Cl_2$ | H |
| 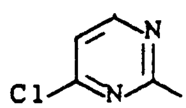 | O | $3,5\text{-}CH_3$ | H |

| $R^1$ | X | $R^2$ | $R^3$ |
|---|---|---|---|
| 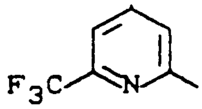 | O | 3,5-$Cl_2$ | H |
| 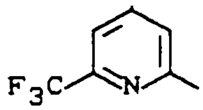 | O | 3,5-$CH_3$ | H |
| 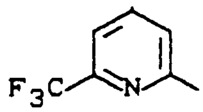 | O | 3,5-$CH_3$ | H |
| 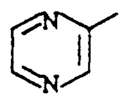 | O | 3,5-$Cl_2$ | H |

Die im folgenden beschriebenen Verfahren sind Gegenstand der älteren nicht veröffentlichen deutschen Patentanmeldung P 320 0373.6.

Setzt man bei dem Verfahren a) zur Herstellung der Verbindungen der Formel II, in welcher $R^3$ nicht für Wasserstoff steht, als Verbindung der Formel Ia 2-[4-(2'-Pyridiloxy)phenyl]1,2,4-triazin-3,5-(2H,4H)-dion und als Verbindung der Formel II Methyliodid ein, läßt sich das Verfahren durch folgendes Schema beschreiben:

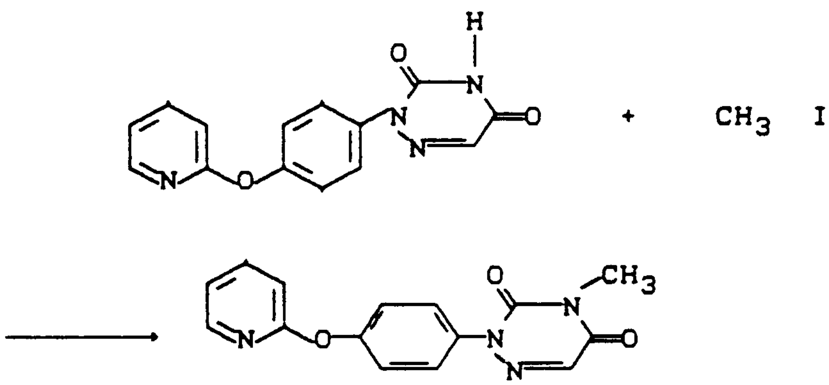


Die Verbindungen der Formel Ia werden wie bei Verfahren b) beschrieben dargestellt.

Die Verbindungen der Formel II sind bekannt oder lassen sich nach bekannten Methoden darstellen. Besonders genannt sei Methyliodid, Ethylbromid.

Das Verfahren wird durchgeführt, indem man eine Verbindung der Formel Ia in Gegenwart einer Base und eines Verdünnungsmittels mit Verbindungen der Formel II umsetzt.

Als Verdünnungsmittel kommen dabei praktisch alle inerten organischen Lösungmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Das Verfahren wird in Gegenwart von Basen durchgeführt. Als bevorzugte Basen seien genannt die Alkalihydroxide wie Natriumyhdroxid, Alkalialkoholate wie Natriummethylat oder Kaliumbutanolat, Metallhydride wie Natriumhydrid oder organische Basen wie 1,8-Diazabicyclo[5,40]-undec-7-en (DBU).

Das Verfahren wird durchgeführt bei Normaldruck und Temperaturen zwischen 20° und 140°C.

Die Reaktion wird durchgeführt indem man äquimolare Mengen der Verbindung der Formel Ia und Base zusammengibt, dieses Gemisch mit einer äquimolaren Menge der Verbindung der Formel II versetzt und auf die Reaktionstemperatur erhitzt.

Nach dem im folgenden beschriebenen Verfahren Ib) lassen sich sowohl die Verbindungen der Formel I als auch die Verbindungen der Formel Ia herstellen.

Wird bei Verfahren Ib) zur Herstellung der Verbindungen der Formel I als Verbindung der Formel III 2-(3-Methyl-4-pyridylphenyl)-1,2,4-triazin-2,5(2H,4H)dion-6-carbonsäure eingesetzt, läßt sich das Verfahren durch folgendes Schema beschreiben:

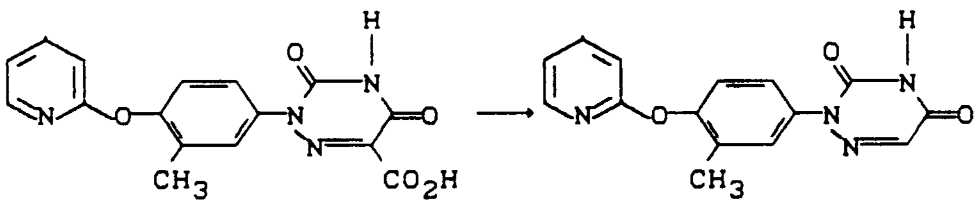

Die Verbindungen der Formel III sind Gegenstand der bereits genannten nicht veröffentlichten Anmeldung der Anmelderin. Es werden bevorzugt Verbindungen der Formel III eingesetzt, in denen X, $R^1$, $R^2$, $R^3$ die bei den Verbindungen der Formel I angegebenen bevorzugten Bedeutungen haben.

Als Einzelverbindungen der Formel III seien genannt:

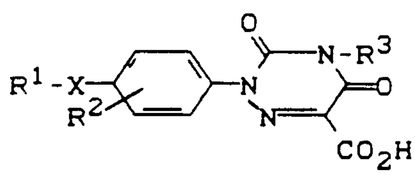

| $R^1$ | X | $R^2$ | $R^3$ |
|---|---|---|---|
| 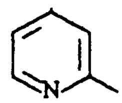 | CH(CN) | 3,5-$Cl_2$ | H |
| " | O | 3,5-$Cl_2$ | H |
| " | O | 3-$CH_3$ | H |
| 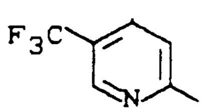 | O | 3,5-$Cl_2$ | H |
| 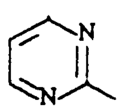 | O | 3,5-$Cl_2$ | H |
| 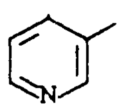 | CH(CN) | 3,5-$Cl_2$ | H |

Die Decarboxylierung wird gegebenenfalls in Gegenwart von inerten organischen Verdünnungsmitteln durchgeführt. Hierzu gehören aliphatische und aromatische, gegebenenfalls halogenierte Kohlenwasserstoffe wie Nonan, Decan, Dodecan, Xylole, Alkohole wie Diethylenglykol, Ether wie Ethylenglykolmonobutylether, Diethylenglykoldibutylether, Sulfoxide wie Dimethylsulfoxid und Sulfone wie Tetramethylensulfon.

Darüber hinaus kann die Reaktion in Gegenwart von mercaptogruppenhaltigen Carbonsäuren wie z.B. Mercaptoessigsäure oder Thiosalicylsäure durchgeführt werden.

Die Umsetzung erfolgt bei Temperaturen zwischen 150 und 300 ° C, gegebenenfalls in Gegenwart von mercaptogruppenhaltigen Carbonsäuren wie z.B. Mercaptoessigsäure vorzugsweise zwischen 160 und 250 ° C, insbesondere zwischen 180 und 210 ° C.

Es wird bei Normaldruck gearbeitet. Die Verbindungen der Formeln III werden in Substanz oder im jeweiligen Verdünnungsmittel, gelost oder suspendiert, erhitzt.

Setzt man im Verfahren Ic) zur Herstellung der Verbindungen der Formel I als Verbindung der Formel IV 1-[3,5-Dichlor-4-(2-pyridyl)-phenyl]-3,3-dimethyl-1,2,4-triazolidin-5-on ein, läßt sich das Verfahren durch folgendes Schema beschreiben:

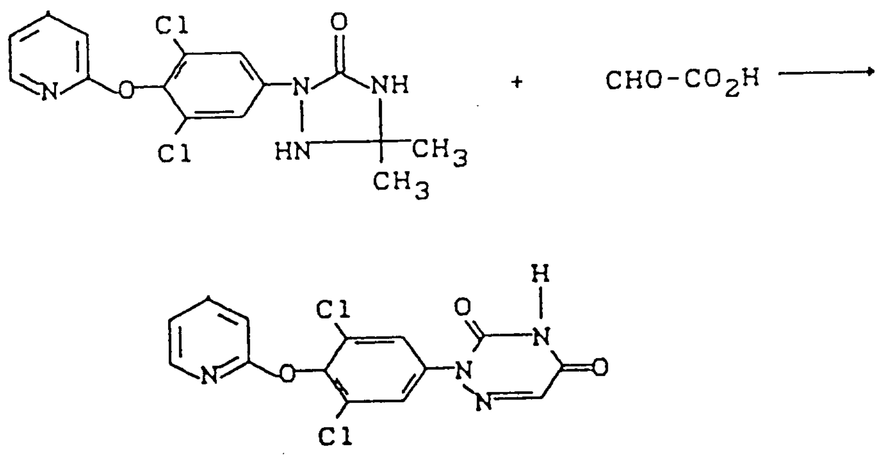


Die Verbindungen der Formel (IV) sind neu. Ihre Herstellung ist Gegenstand der bereits genannten nicht veröffentlichten Anmeldung der Anmelderin.

Bevorzugt werden Verbindungen der Formel IV eingesetzt, in denen X, $R^1$, $R^2$ die bei den Verbindungen der Formel I angegebenen bevorzugten Bedeutungen haben und $R^6$ und $R^7$ unabhängig voneinander für $C_{1-4}$-Alkyl, insbesondere Methyl oder Ethyl stehen. Im einzelnen seien genannt:

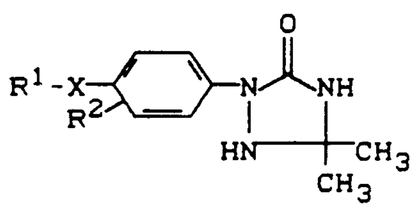

| $R^1$ | X | $R^2$ |
|---|---|---|
| 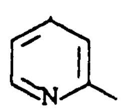 | O | 3,5-$Cl_2$ |
| 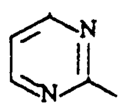 | O | 3,5-$Cl_2$ |
| 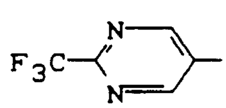 | O | 3,5-$Cl_2$ |

Das Verfahren wird durchgeführt, indem man eine Verbindung der Formel IV in einem Verdünnungsmittel in Gegenwart von Glyoxylsäure und einer katalytischen Menge konzentrierter Mineralsäure erwärmt und das Rohprodukt nach wäßriger Aufarbeitung an Kieselgel chromatographiert.

Als Verdünnungsmittel können alle inerten organischen Lösungsmittel verwendet werden, die z.B. bei Verfahren Ia) angeführt sind. Als Mineralsäure wird vorzugsweise Schwefelsäure verwendet. Es wird bei Temperaturen zwischen 60 °C und 130 °C, vorzugsweise bei 100 °C, gearbeitet.

Wird im Verfahren d) zur Herstellung der Verbindungen der Formel I als Verbindung der Formel V Ethyl-N-[[3-methyl-4-(5'-trifluoromethyl-2'-pyridinyloxyphenyl] hydrazinyliden-carbonyl]carbamat eingesetzt, läßtsich das Verfahren durch folgendes Schema beschreiben:

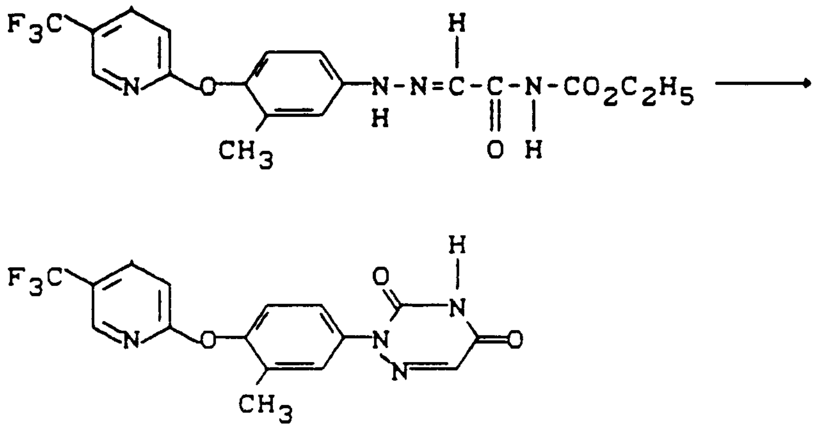

Die Verbindungen der Formel V sind neu. Ihre Herstellung ist Gegenstand der bereits erwähnten nicht veröffentlichten Anmeldung der Anmelderin.

Bevorzugt werden Verbindungen der Formel V eingesetzt, in denen X, $R^1$, $R^2$, $R^3$ die bei den Verbindungen der Formel I angegebenen bevorzugten Bedeutungen haben, $R^8$ für $C_{1-4}$-Alkyl, insbesondere Methyl oder Ethyl und Phenyl steht und $R^9$ für Wasserstoff oder CN steht.

Als Einzelverbindungen der Formel V seien genannt:

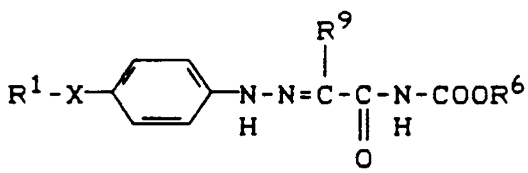

| $R^1$ | X | $R^2$ | $R^4$ | $R^6$ |
|---|---|---|---|---|
| 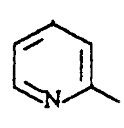 | CHCN | 3,5-$Cl_2$ | H | Et |
| | O | 3,5-$Cl_2$ | H | Et |
| | O | 3-$CH_3$ | H | Et |
| 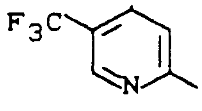 | O | 3,5-$Cl_2$ | H | Et |
| 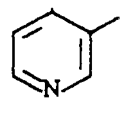 | CHCN | 3,5-$Cl_2$ | H | Et |

Das Verfahren wird durchgeführt, indem man eine Verbindung der Formel V erhitzt, gegebenenfalls in Gegenwart eines Lösungsmittels und einer Base.

Als Lösungsmittel und Basen finden die bei Verfahren a) zur Herstellung der Verbindungen I aufgeführten Lösungsmittel und Basen Verwendung. Als weitere besonders bevorzugte organische Lösungsmittel werden Alkohole wie z.B. Ethanol oder organische Säuren wie z.B. Eisessig eingesetzt.

Besonders bevorzugte Basen sind die Hydroxide und Acetate der Alkali- oder Erdalkalimetalle wie z.B. NaOH oder Natrium- und Kaliumacetat.

Die Umsetzung erfolgt unter Normaldruck bei Temperaturen zwischen 70 und 150 °C, vorzugsweise zwischen 70 und 100 °C.

Die verwendete Base wird in 10-80 %igem molarem Überschuß eingesetzt. Das Reaktionsgemisch wird nach abgeschlossener Cyclisierung vorzugsweise mit einer verdünnten Mineralsäure wie z.B. Salzsäure angesäuert und das als Feststoff anfallende Produkt abfiltriert.

Setzt man bei dem Verfahren Ie) als Verbindung VI 2-(3,5-Dichloro-4-hydroxyphenyl)-1,2,4-triazin-3,5-(2H,4H)-dion und als Verbindung der Formel VII 2-Chlorpyridin ein und läßt sich das Verfahren durch folgendes Formelschema beschreiben.

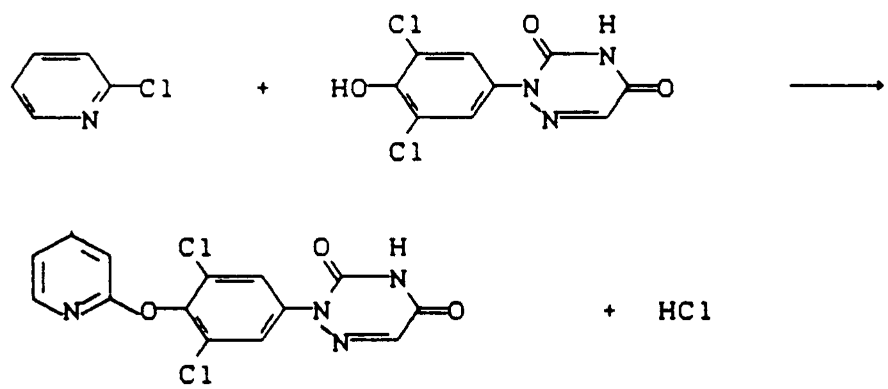

Verbindungen der Formel VI
in welcher
$R^2$ und $R^3$ für Wasserstoff stehen, sind bekannt (J. Slouka, Acta Unio Palacki Olomuk. Fac. Rerum. Nat. 1984 (Chem 23), 39 - 45; C.A. 102 203946c).

Andere Verbindungen der Formel VI sind Gegenstand einer bislang nicht veröffentlichen Ameldung der Anmelderin (Deutsche Patentanmeldung P 3 805 660). Sie lassen sich analog zu den für die Verbindungen der Formel I angegebenen Verfahren herstellen.

Bevorzugt seien Verbindungen der Formel VI genannt, in denen $R^2$ und $R^3$ die bei den Verbindungen der Formel I genannten bevorzugten Bedeutungen haben.

Die substituierten Heterocyclen der Formel VII sind bekannt oder lassen sich analog zu bekannten Verfahren herstellen (z.B. Beilstein Bd. 20, S. 230, 20, 1. Erg. S. 82, 20, S. 359; Katrizky und Rees, Comprehensive Het. Chem. Col. 6 1984).

Sie besitzen die weiter oben bei den Verbindungen der Formel I angegebenen bevorzugten Bedeutungen. Im einzelnen seien die folgenden Verbindungen der Formel VII genannt:

**VII**

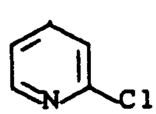 , 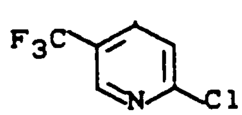 , 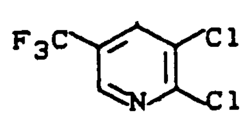

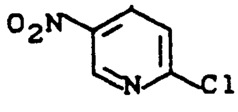 , 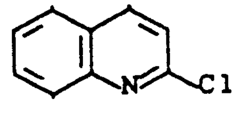

Die Reaktion wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid.

Die Reaktion wird in Gegenwart von anorganischen oder organischen Säureakzeptoren durchgeführt.

Als solche seien z.B. genannt:
Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Umsetzung erfolgt bei Temperaturen zwischen 50 und 200 °C, vorzugsweise zwischen 80 und 160 °C bei Normaldruck oder erhöhtem Druck. Es wird bevorzugt bei Normaldruck gearbeitet.

Das Verfahren wird durchgeführt, indem äquimolare Mengen der Verbindungen der Formel XVII und XVIII in einem der angegebenen Verdünnungsmittel zusammengegeben und erhitzt werden. Nach vollendeter Umsetzung wird das Reaktionsgemisch mit verdünnter anorganischer Säure (z.B. Salzsäure) angesäuert und der entstehende Niederschlag abfiltriert, gewaschen und getrocknet.

Die Wirkstoffe eignen sich bei günstiger Warmblütertoxizität zur Bekämpfung von parasitischen Protozoen von Mensch und Tier die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den parasitischen Protozoen zählen:
Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. irresidua, E. labbeana, E. leucarti, E. magna, E. maxima, E. media, E. meleagridis, E. meleagrimitis, E. mitis, E. necatrix, E. ninakohlyakimovae, E. ovis, E.parva, E.pavonis, E.perforans, E. perforans, E. phasani, E. piriformis, E. praecox, E. residualis, E.scaora, E. spec., E. stiedai, E. suis, E. tenella, E. truncata, E. truttae, E. zuernii, Globidium spec., Isospora belli, I. canis, I. felis, I. ohioensis, I.rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozytoz z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B.bigemina, B.bovis, B. canis, B. spec., Theileria parva, Theileria spec., wie Adeleina z.B. Hepatozoon canis, H. spec.

Ferner Myxosporidia and Microspora, for example Glugea spec.

Ferner Pneumocystis carinii, sowie Ciliophora (Ciliata) wie z.B. Balantidium coli, Ichthiophthirius spec., Trichodina spec., Epistylis spec.

Die erfindungsgemäßen Verbindungen sind auch wirksam gegen Protozoen, die als Parasiten bei Insekten auftreten. Als solche seien genannt Parasiten des Stammes Microsporidia insbesondere der Gattung Nosema. Besonders genannt sei Nosema apis bei der Honigbiene.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z.B. Plagioscion, Channel catfish. Besonders geeignet sind die erfindungsgemäßen Mittel zur Behandlung von Fischbrut, z.B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern, Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgestpritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$,Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-$\beta$-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt; Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:
Für die Heilung und Prophylaxe etwa der Coccidiose bei Geflügel, insbesondere bei Hühnren, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeitieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesodnere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Darüber hinaus sind die erfindungsgemäßen Verbindungen wirksam gegenüber verschiedenen zu den Helminthen (Würmern) zählenden Fischparasiten.

Zu den Parasiten bei Fischen gehören aus dem Unterreich der Protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Pleistophora spp.,

aus dem Stamm der Plathelminthen: Trematoden; Monogenea z.B. Dactylogyrus app., Gyrodactylus spp., Pseudodactylogyrus spp., Diplozoon spp., Cestoden, z.B. aus den Gruppen der Caryphyllidea (z.B. Caryophyllaeus laticeps), Pseudophyllidea (z.B. Diphyllobothrium spp.), Tetraphyllidea (z.B. Phyllobothrium spp.) und Protocephalida (z.B. Arten der Gattung Proteocephalus) und aus dem Stamm der Arthropoda verschiedene parasitische Crustaceen, insbesondere aus den Unterklassen der Branchiura (Fischläuse) und Copepoda (Ruderfußkrebse) sowie den Ordnungen der Isopoda (Asseln) und Amphipoda (Flohkrebse).

Die Behandlung der Fische erfolgt entweder oral, z. B. über das Futter oder durch Kurzzeitbehandlung, "medizinisches Bad", in das die Fische eingesetzt und in dem sie eine Zeitlang (Minuten bis mehrere Stunden) z. B. beim Umsetzen von einem Zuchtbecken zum anderen gehalten werden.

Es kann aber auch eine vorübergehende oder dauernde Behandlung des Lebensraums der Fische (z. B. ganzer Teichanlagen, Aquarien, Tanks oder Becken), in denen die Fische gehalten werden, erfolgen.

Der Wirkstoff wird in Zubereitungen verabreicht, die den Anwendungen angepaßt sind.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teichbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7-10, vorzugsweise aber einen pH-Wert von 8-10 haben.

Die Konzentration des Wirkstoffes kann im Bereich von 0,5-50 % liegen, vorzugsweise aber in einem Bereich von 1-25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethyalkohol, Isopropylalkohol, Benzylalkohol, Glycerin, Propylenglykol, Polyethylenglykole, Poly(oxoethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D,L-Arginin, L- bzw. D, L-Lysin, Methylglucosamin, Glucosamin, 2-Amino-2-hydroxymethylpropandiol-(1,3) ferner wie N,N,N',N'-tetrakis-(2-hydroxypropyl)-ethylendiamin oder Polyether-Tetrol auf der Basis Ethylendiamin (M.G. 480-420), anorganische Basen, wie Ammoniak oder Natriumcarbonat-gegebenenfalls unter Zugabe von Wasser.

Die Zubereitungen können auch 0,1 bis 20 Gew.-%, vorzugsweise 0,1-10 Gew.-% anderer Formulierhilfsstoffe, wie Antioxydantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2-50 mg Wirkstoff pro Liter Wasser bevorzugt 5-10 mg pro Liter, bei einer Behandlungsdauer von 3-4 Stunden. Bei der Behandlung von jungen Karpfen wird z.B. mit einer Konzentration von 5-10 mg/l und einer Behandlungsdauer von ca. 1-4 Stunden gearbeitet.

Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1-5 mg Wirkstoff pro Liter Wasser verwendet werden.

Zubereitungen zur Anwendung als Futterzusatz sind z.B. wie folgt zusammengesetzt;

a)

| | |
|---|---|
| Wirkstoff der Formel I<br>Sojabohnen-Protein | 1 - 10 Gewichtsteile<br>49 - 90 Gewichtsteile |

b)

| | |
|---|---|
| Wirkstoff der Formel I<br>Benzylalkohol<br>Hydroxypropylmethylcellulose<br>Wasser | 0,5 - 10 Gewichtsteile<br>0,08 - 1,4 Gewichtsteile<br>0 - 3,5 Gewichtsteile<br>Rest ad 100 |

Zubereitungen zur Anwendung bei "medizinischen Bädern" und zur Teichbehandlung sind z.B. wie folgt zusammengesetzt und hergestellt.

c) 2,5 g Wirkstoff der Formel (I) werden in 100 ml Triethanolamin unter Erwärmen gelöst.

d) 2,5 g Wirkstoff der Formel (I)

12,5 g Milchsaure werden in 100 ml Triethanol amin unter Erwärmen und Rühren gelöst.

e) 10,0 g Wirkstoff der Formel (I) wird in 100 ml Monoethanolamin gelöst.

f)

| | |
|---|---|
| Wirkstoff der Formel I<br>Propylenglykol<br>Natriumcarbonat<br>Wasser | 5,0 g<br>50,0 g<br>5,0 g<br>ad 100 ml |

g)

| | |
|---|---|
| Wirkstoff der Formel I<br>Monoethanolamin<br>N-Methylpyrrolidon | 5,0 g<br>10 g<br>ad 100 ml |

h)

| | |
|---|---|
| Wirkstoff der Formel I<br>Natriumcarbonat<br>Polyethylenglykol200 | 2,5 g<br>5,0 g<br>ad 100 ml |

Der Wirkstoff wird unter Erwärmen im Polyethylenglykol gelöst und Natriumcarbonat darin suspendiert.

Beispiel A

Coccidiose bei Hühnern

9 bis 11 Tage alte Küken wurden mit 40000 sporulierten Oozysten von stark virulenten Stämmen von Eimeria acervulina, E. maxima und E. tenella, den Krankheiteserregern der intestinalen Coccidiose infiziert.

Von 3 Tage vor der Infektion bis 8 Tage nach der Infektion (Ende des Versuchs) wurde Wirkstoff in der angegebenen Konzentration im Futter der Tiere eingemischt verabreicht.

Die Zahl der Oozysten im Kot wurde mit Hilfe der McMaster-Kammer bestimmt (siehe Engelbrecht und Mitarbeiter "Parasitologische Arbeitsmehoden in Medizin und Veterinärmedizin", S. 172, Akademie-Verlag, Berlin (1965)).

Als wirksam werden diejenigen Dosen angesehen, die die Ausscheidung von Oozysten und/oder klinische Symptome der Coccidiose einschließlich der Mortalität vollständig oder in hohem Maße verhüten. In der folgenden Tabelle werden die wirksamen Dosen angegeben:

Tabelle 1

Coccidiose bei Hühnern

| Beispiel Nr. | Dosis ppm. | Sterberate tot/eingesetzt | Oocystenausscheidung in % im Vergleich zur unbehandelten infizierten Kontrolle | Gewichtszunahme in % im Vergleich zur nicht infizierten unbehandelten Kontrolle | Blutausscheidung mit dem Kot |
|---|---|---|---|---|---|
| unbehandelte infizierte Kontrolle | | 2/9 | 100 | 40 | stark |
| 9 | 50 | 0/3 | 0 | 100 | keine |
| | 25 | 0/3 | 0 | 100 | keine |
| | 10 | 0/3 | 0,3 | 100 | keine |

Beispiel B

Eimeria falcifor mis/Maus-Test

Labormäuse mit ca, 18 g Körpergewicht werden pro Tier künstlich mit ca. 18 000 spooulierten Oocysten von Ei eria falciformis infiziert. Die infizierten Tiere werden dann am 1., 2., 3., 6., 7., 8. Tag nach der Infektion behandelt.

Dabei wird jedem Tier 0,5 ml Wasser in den dem die angegebene Dosis Wirkstoff gelost oder suspendiert ist pro Schlundsonde verabreicht. Pro Dosis werden 4 Tiere behandelt. 9 Tage nach Infektion im Darm sowie beobachtee klinische Symptome und Mortalität während des Versuchs werden zur Beurteilung der Wirksamkeit herangezogen. Der Grad der Wirksamkeit wird wie folgt beurteilt:
gut wirksam = 2
gering wirksam = 1
nicht wirksam = 0
Kontrolle: bei den unbehandelten Kontrolltieren,
komnmt es ab dem 7. Tag nach Infektion zu einer massiven ausscheidung, zu blutigen Durchfällen und zu einer infektionsbedingten Herblichkeit von ca. 30 % der Tiere.

Tabelle 2

| Wirkstoff Beispiel-Nr. | Dosis mg/kg Körpergewicht | Wirksamkeit nach 9 Tagen |
|---|---|---|
| Kontrolle | - | 0 |
| 9 | 50 | 2 |
| | 10 | 2 |
| | 5 | 2 |
| | 50 | 2 |
| | 10 | 2 |
| 12 | 5 | 2 |
| | 1 | 2 |
| | 0,5 | 2 |

Analog werden hergestellt;

Beispiel 1

2-[4-(2-Pyridyloxy)phenyl]-3-N-methyl-3-N-methyl-3,5-(2H,4H)-dioxo-1,2,4-triazin

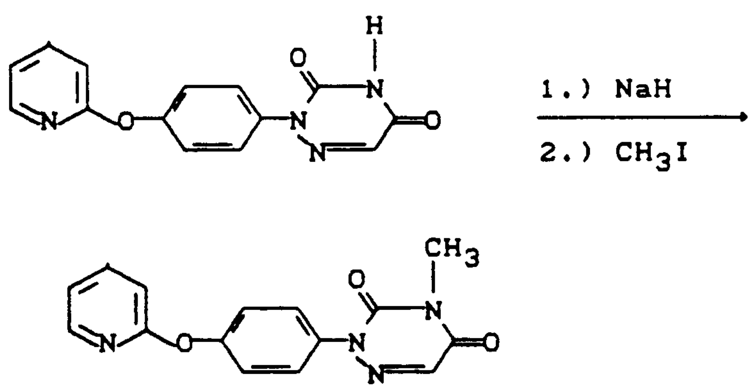


2 g (7 mmol) Pyridinyloxyarylazauracil werden in 20 ml absolutem DMSO gelöst und mit 0,16 g (6 mmol) Natriumhydrid versetzt. Man rührt 20 min bei RT und gibt dann 1,5 g (9 mmol) Methyljodid in 5 ml DMSO unter Argon zu. Man erwärmt auf 50 °C und hält 3 h bei dieser Temperatur. Anschließend wird das Reduktionsgemisch i. V. eingeengt und dann mit Wasser versetzt. Nach dem Absaugen des ausgefallenen

Feststoffs erhält man so 1,5 g (72 % der Theorie) der N-Methylverbindung.

Beispiel 2

2-(2-Pyridyloxyphenyl)-1,2,4-triazin-3,5(2,4H)dion

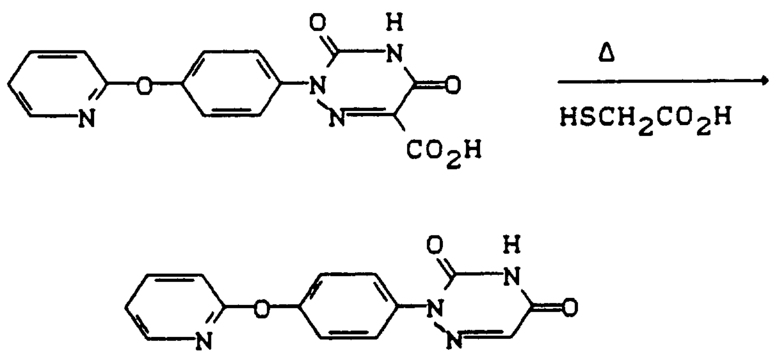


10 g (0,03 mol) Carbonsäure werden in 20 ml Mercaptoessigsäure auf 170 °C erhitzt. Nach 1,5 h läßt man abkühlen, versetzt mit Wasser und erhält nach Abfiltration 27 g (82 % der Theorie) decarboxyliertes Produkt.

Beispiel 2a

2-(4-(2-pyridinyloxy)-3,5-(2H,4H),dioxo-6-cyano-1,2,4-triazin

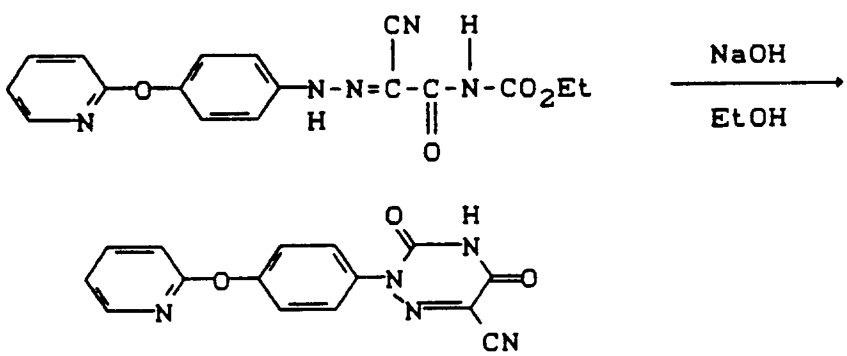


12 g (0,034 mol) des Hydrazonocyanurethans, 1,8 g (0,44 mol) NaOH werden in 50 ml abs. Ethanol 2 h unter Rückfluß erhitzt. Anschließend kühlt man ab, säuert mit Salzsäure an und engt i.V. ein. Man verrührt mit Wasser und saugt den ausgefallenen Niederschlag ab, Man erhält so nach Trocknung 8,9 g (85 % der Theorie) Cyanazauracil aus dem durch Hydrolyse die entsprechende Azauracilcarbonsäure entsteht.

Beispiel 3

1-[3,5-Dichloro-4-(2-pyridyl)-phenyl]-1,2,4-triazin-3,5-(2H,4H)-dion

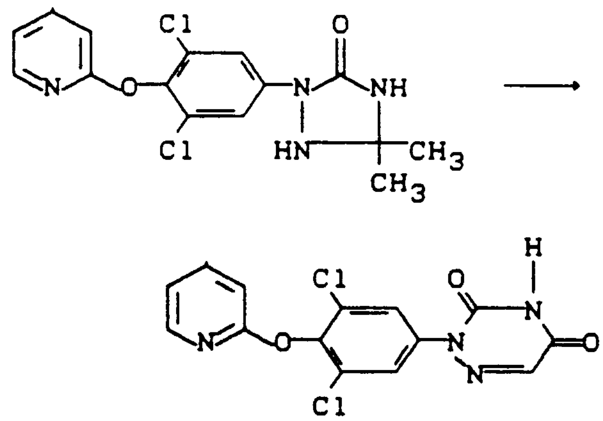

3 g (8,5 mmol) 1-[3,5-Dichloro-4-(2-pyridyl)-phenyl]-3,3-dimethyl-1,2,4-triazolidin-5-on werden in 50 ml Dioxan gelöst und mit 0,78 g (8,5 mmol) Glyoxylsäuremonohydrat und 0,1 ml $H_2SO_4$ konz. versetzt. Man rührt zunächst bei Raumtemperatur 2 h und gibt dann weitere 0,8 g Glyoxylsäure zu. Nach 5 h Rühren unter Rückfluß gießt man das Reaktionsgemisch auf Wasser und extrahiert 3 x mit Ethylacetat. Das Lösungsmittel wird im Vakuum abgezogen und der Rückstand mit Dichlormethan/Methanol (95:5) an $SiO_2$ chromatographiert. Man erhält so 1,2 g (40 % d. Th.) des entsprechenden Azauracils.

Beispiel 4

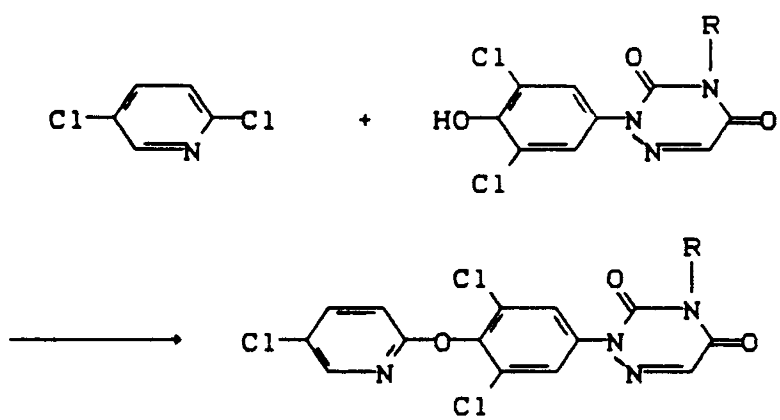

29 g (0,01 mol) Hydroxyphenylazauracil, 1,2 g (0,01 mol) Dichlorpyridin und 1,4 g (0,01 mol) Kaliumcarbonatwerden in 20 ml trockenem DMF unter Rückfluß 2 h gerührt. Das abgekühlte Reaktionsgemisch wird mit HCl angesäuert und ausgefallenes Produkt abgesaugt. Nach dem Umkristallisieren aus Ethanol erhalt man 2,9 g (78 % der Theorie) Pyridinyloxarylazaurazil.

Nach analogen Verfahren können die folgenden Beispiele hergestellt werden:

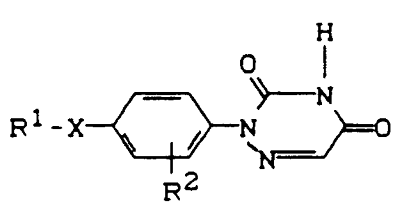

| Bsp.-Nr. | $R^1$ | X | $R^2$ | $R^3$ | Fp. [°C] |
|---|---|---|---|---|---|
| 5 | 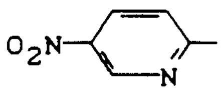 | O | H | H | 262 |
| 6 | 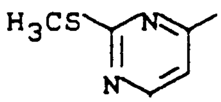 | O | H | H | 168 |
| 7 | 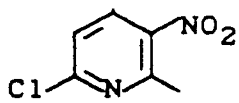 | O | 3,5-$Cl_2$ | H | 231 |
| 8 | 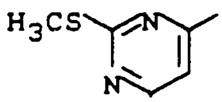 | O | 3,5-$Cl_2$ | H | 185 |
| 9 | 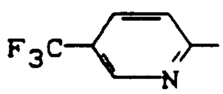 | O | 3,5-$Cl_2$ | H | 191 |

| Bsp.-Nr. | $R^1$ | X | $R^2$ | $R^3$ | Fp. [°C] |
|---|---|---|---|---|---|
| 10 | 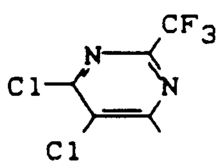 | O | 3,5-$Cl_2$ | H | 201 |
| 11 | 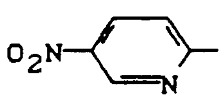 | O | 3,5-$Cl_2$ | H | 221 |
| 12 | 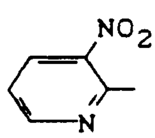 | O | 3,5-$Cl_2$ | H | 205 |
| 13 | 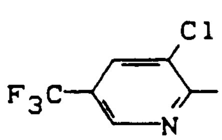 | O | 3,5-$Cl_2$ | H | 208 |
| 14 | 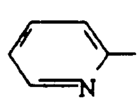 | O | 3,5-$Cl_2$ | H | 223 |

## Patentansprüche

1. Verwendung von 1,2,4-Trizin d ione der allgemeinen Formel (I)

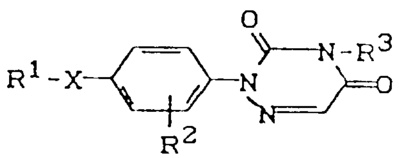

I

in welcher
   $R^1$ für gegebenenfalls durch Halogen, Alkyl, Cyano, Nitro, Alkoxy, Alkylthio, Halogenalkyl; Halogenalkoxy, Halogenalkylthio, Halogensulfonyl, Halogenalkylsulfinyl, Amino, Alkylamino, Halogenalkylamino, Acylamino substituiertes Pyridyl, Pyrazinyl oder Pyrimidinyl steht,
   X für O, S, SO, $SO_2$ oder $-CR^4(CN)-$ steht,
   $R^2$ für Wasserstoff einen oder mehrere, gleiche oder verschiedene Reste der Gruppe Wasserstoff, Halogen, Nitro, Alkyl, Alkoxy, Alkylthio, Halogenalkyl, Halogenalkoxy, Alkylthio, Halogenalkylthio, steht,
   $R^3$ für Wasserstoff,
   Alkyl, Alkenyl, Alkinyl, Aralkyl steht,
   $R^4$ für Wasserstoff oder Alkyl steht,
zur Herstellung eines Medikaments zur Bekämpfung parasitärer Protozoen.

2. Mittel gegen parasitäre Protozoen, gekennzeichnet durch einen Gehalt an mindestens einem substituierten 1,2,4-Triazindione der Formel (I) gemäß Anspruch 1.

3. Verwendung nach Anspruch 1, wobei das Verfahren zur Herstellung von Mitteln gegen parasitäre Protozoen, dadurch gekennzeichnet ist, daß man substituierte 1,2,4-Triazindione der Formel (I) gemäß Anspruch 1 mit Streckmitteln und/oder oberflächenaktiven Mitteln vermischt.

## Claims

1. Use of 1,2,4-triazinediones of the general formula (I)

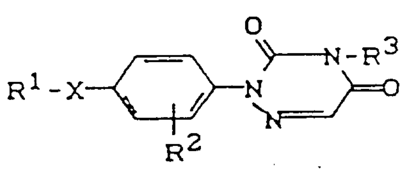

I

in which
   $R^1$ represents pyridyl, pyrazinyl or pyrimidinyl optionally substituted by halogen, alkyl, cyano, nitro, alkoxy, alkylthio, halogenoalkyl, halogenoalkoxy, halogenoalkylthio, halogenoalkylsulphonyl, halogenoalkylsulphinyl, amino, alkylamino, halogenoalkylamino or acylamino,
   X represents O, S, SO, $SO_2$ or $-CR^4(CN)-$,
   $R^2$ represents hydrogen or one or more identical or different radicals of the group comprising hydrogen, halogen, nitro, alkyl, alkoxy, alkylthio, halogenoalkyl, halogenoalkoxy, alkylthio and halogenoalkylthio,
   $R^3$ represents hydrogen, alkyl, alkenyl, alkinyl or aralkyl and
   $R^4$ represents hydrogen or alkyl,
for the preparation of a medicament for combating parasitic protozoa.

2. Agent against parasitic protozoa, characterized in that it contains at least one substituted 1,2,4-triazinedione of the formula (I) according to Claim 1.

**3.** Use according to Claim 1, the process for the preparation of agents against parasitic protozoa being characterized in that substituted 1,2,4-triazinediones of the formula (I) according to Claim 1 are mixed with extenders and/or surface-active agents.

## Revendications

**1.** Utilisation de 1,2,4-triazine-diones de formule générale (I)

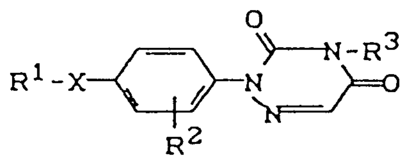

I

dans laquelle

- $R^1$ représente un groupe pyridyle, pyrazinyle ou pyrimidinyle éventuellement substitué par un halogène, un radical alkyle, cyano, nitro, alkoxy, alkylthio, halogénalkyle, halogénalkoxy, halogénalkylthio, halogénosulfonyle, halogénalkylsulfinyle, amino, alkylamino, halogénalkylamino, acylamino,
- X représente O, S, SO, $SO_2$ ou $-CR^4(CN)-$,
- $R^2$ est de l'hydrogène ou représente un ou plusieurs restes, identiques ou différents, du groupe hydrogène, halogène, nitro, alkyle, alkoxy, alkylthio, halogénalkyle, halogénalkoxy, alkylthio, halogénalkylthio,
- $R^3$ est de l'hydrogène, un groupe alkyle, alcényle, alcynyle, aralkyle,
- $R^4$ est de l'hydrogène ou un groupe alkyle,

pour la préparation d'un médicament destiné à la lutte contre des protozoaires parasites.

**2.** Composition destinée à combattre des protozoaires parasites, caractérisée en ce qu'elle contient au moins une 1,2,4-triazinedione substituée de formule (I) suivant la revendication 1.

**3.** Utilisation suivant la revendication 1, dans laquelle le procédé de préparation de compositions contre des protozoaires parasites est caractérisé en ce qu'on mélange des 1,2,4-triazinediones substituées de formule (I) suivant la revendication 1 avec des diluants et/ou avec des agents tensio-actifs.